# EUROPEAN PATENT APPLICATION

(11) **EP 4 116 430 A1**
(43) Date of publication of application: **11.01.2023**
(21) Application number: 21763688.5
(22) Date of filing: 04.03.2021
(51) Int. Cl.: C12Q 1/68, C12N 15/09, C12N 15/11

(54) **METHOD FOR DETECTING RANDOM OFF-TARGET EFFECT OF SINGLE-BASE EDITING SYSTEM**

(30) Priority: 04.03.2020 CN 202010144167
(71) Applicant: Suzhou Qi Biodesign biotechnology Company Limited, Jiangsu 215127 (CN)
(72) Inventor: GAO, Caixia, Beijing 100101 (CN); JIN, Shuai, Beijing 100101 (CN); ZHU, Zixu, Beijing 100101 (CN); FEI, Hongyuan, Beijing 100101 (CN)
(74) Representative: Michalski Hüttermann & Partner Patentanwälte mbB
(86) International application number: PCT/CN2021/079082
(87) International publication number: WO 2021/175287

(57) **Abstract**

The present invention belongs to the field of gene editing, and particularly relates to a method and means for detecting genome wide random off-target effect of a base editing system in a rapid and high-throughput mode.

## Description

### Technical Field

The present invention belongs to the field of gene editing, and particularly relates to a method for detecting genome-wide random off-target effect of a base editing system in a rapid and high-throughput mode.

### Background Art

Genome editing technology is a genetic engineering technology for conducting targeted modification on a genome based on artificial nucleases, and plays a more and more powerful role in agricultural and medical research. Clustered regularly interspaced short palindromic repeats/CRISPR associated (CRISPR) systems are the most widely-used genome editing tools at present, and Cas protein can target any position in the genome under the guiding effect of artificially-designed guide RNA.

Base editing systems are a novel gene editing technology developed based on the CRISPR system and can be divided into cytosine base editing systems and adenine base editing systems. Cytosine deaminase and adenine deaminase are fused with Cas9 single-stranded nickase, and under the targeting effect of guiding RNA, the Cas9 single-stranded nickase generates a single-stranded DNA region, so that deaminase can efficiently remove amino groups from C or A nucleotides on single-stranded DNA at the targeting position to obtain U bases and I bases, and then the bases are repaired into T bases or G bases in the cell self-repairing process. However, it is found that the cytosine base editing system will cause an unpredictable genome-wide off-target phenomenon, which is possibly caused by random deamination phenomenon occurred in a high-transcription active region in a genome due to excessive expression of cytosine deaminase in the genome, and the genome-wide off-target phenomenon greatly affects the applications of the cytosine base editing system.

So far, the only method for evaluating the off-targets of the base editing system is a genome-wide sequencing technology which sequence a large number of cells or biological individuals subjected to base editing and count the point mutation number in the genome-wide range so as to evaluate the random off-target effect of the base editing system in the genome range, but this method is cost and time inefficient and cannot detect the genome-wide random off-target effect of various base editing systems in a high-throughput mode.

There is still the need of a simple and low-cost method for detecting the random off target effect of base editing systems.

### Summary of the Invention

The inventor finds that co-transferring the base editing system to be identified with another CRISPR system which can generate a single-stranded region and is orthogonal to the base editing system into the cell can generate a long-term stable single-stranded region in the genome, thus the base editor which can randomly act on a single-stranded DNA region deaminates on the target bases of the single-stranded region, and the random off-target effect of the base editing system can be efficiently, simply and conveniently detected by amplicon high-throughput sequencing. Such method is called as Trans-ssDNA amplicon deep sequencing (TA-AS) method.

### Brief Description of the Drawings

FIG. 1 shows schematic diagram of orthogonal system detection vectors.
FIG. 2 shows verification of TA-AS system by rice protoplast transformation.
FIG. 3 shows the schematic diagram of a BE3 vector.
FIG. 4 shows detecting off target effect of different base editing systems by TA-AS method.
FIG. 5 shows genome-wide sequencing method for detecting off target effect of five base editing systems.
FIG. 6 shows a regression analysis of the genome-wide sequencing results and the TA-AS results.

### Detailed Description of the Invention

In one aspect, the present invention provides a method for detecting random off-target effect of a base editing system, the method comprises the following steps:
a) introducing a base editing system to be detected into a cell or an organism;
b) introducing a CRISPR detection system which targets at least one detection target site in the genome into the cell or the organism, wherein the CRISPR detection system being capable of forming a single-stranded DNA region at the at least one detection target site, and the guide RNA of the CRISPR detection system being incompatible with the guide RNA of the base editing system to be detected;
c) extracting nucleic acid from the cell or the organism, amplifying the sequence of the at least one detection target site, and sequencing the amplicons; and
d) determining nucleotide mutation in the at least one detection target site.

In some embodiments, the detection of a nucleotide mutation in the at least one detection target site indicates that the base editing system to be detected has off target effect. The amount of the nucleotide mutations detected in the at least one detection target site represents the degree of off-target effect, and more nucleotide mutations detected represents higher degree of off-target effect.

The base editing system to be detected can include a base editor to be detected or an expression construct comprising the coding sequence a base editor to be detected, and/or a corresponding guide RNA (gRNA) or an expression construct comprising a coding sequence of the gRNA. In some embodiments, the base editing system to be detected in the step a) only includes a base editor to be detected or an expression construct comprising a coding sequence thereof.

As used herein, the "base editor" refers to a fusion protein containing a CRISPR effector protein and deaminase. According to the different deaminases, the base editors can be divided into cytosine base editors and adenine base editors. In some preferred embodiments, the base editing system to be detected in the present invention comprises a cytosine base editor.

The cytosine base editor is usually a fusion protein containing a CRISPR effector protein and a cytosine deaminase. The cytosine deaminase in the base editor is capable of converting by deamination a cytidine on the single-stranded DNA generated in formation of a CRIPR effector protein-guide RNA-target DNA complex into U, and then base substitution of C to T is achieved through mismatched base repairing. In some embodiments, the cytosine base editor further contains a uracil DNA glycosylase inhibitor (UGI). In cells, the uracil DNA glycosylase inhibitor catalyzes the removal of U from DNA and starts base excision repair (BER), resulting in repair of U: G into C: G. Therefore, without any theoretical limitation, including the uracil DNA glycosylase inhibitor (UGI) in the cytosine base editor can increase the efficiency of C to T base editing.

Examples of cytosine deaminase include, but are not limited to, for example, APOBEC1 deaminase, activation-induced cytidine deaminase (AID), APOBEC3G, CDA1, human APOBEC3A deaminase, or functional variants thereof. In some embodiments, the cytosine deaminase is human APOBEC3A or a functional variant thereof. In some embodiments, the cytosine deaminase is APOBEC1 or a functional variant thereof. In some specific embodiments, the cytosine deaminase includes an amino acid sequence of one of SEQ ID NO: 7-10.

However, the method of the present invention can be used for testing the off-target effect of base editors comprising various cytosine deaminase variants.

As used herein, the term "CRISPR effector protein" generally refers to nuclease existing in a naturally occurring CRISPR system, and modified forms, variants, catalytically active fragments and the like thereof. The term covers any effector protein based on the CRISPR system and capable of achieving gene targeting (such as gene editing and targeted gene regulation) in cells.

Examples of the "CRISPR effector protein" include Cas9 nuclease or a variant thereof. The Cas9 nuclease can be Cas9 nuclease from different species, such as spCas9 from S. *pyogenes* or SaCas9 derived from S. *aureus.* The terms "Cas9 nuclease" and the "Cas9" can be used interchangeably in the present invention, and refer to a RNA-guided nuclease comprising a Cas9 protein or a fragment thereof (such as a protein comprising an active DNA cleavage domain of Cas9 and/or a gRNA binding domain of Cas9). Cas9 is a component of a CRISPR/Cas (Clustered regularly interspaced short palindromic repeats/CRISPR associated) genome editing system, and can target and cleave a DNA target sequence to form a DNA double-strand break (DSB) under the guidance of guide RNA.

The examples of the "CRISPR effector protein" can further comprise Cpfl nuclease or a variant thereof, such as a high-specificity variant. The Cpfl nuclease can be Cpfl nuclease from different species, such as Cpfl nuclease from *Francisella novicida* U112, *Acidaminococcus sp.* BV3L6 and *Lachnospiraceae bacterium* ND2006.

In some embodiments, the CRISPR effector protein of the base editor of the present invention is a nuclease inactivated CRISPR effector protein. In some embodiments, the CRISPR effector protein of the base editor of the present invention is a CRISPR effector protein with nickase activity. In some embodiments, the CRISPR effector protein of the base editor of the present invention is a Cas9 nickase. In some preferred embodiments, the CRISPR effector protein of the base editor of the present invention is a nickase form (nSpCas9) of SpCas9 from S. *pyogenes.* For example, the nSpCas9 comprises an amino acid sequence shown in SEQ ID NO: 1. In some embodiments, the CRISPR effector protein of the base editor of the present invention is a nickase form (nSaCas9) of SaCas9 from *S*. *aureus.* For example, the nSaCas9 comprises an amino acid sequence shown in SEQ ID NO: 2.

The CRISPR detection system of the present invention may comprise a CRISPR effector protein or an expression construct comprising a coding nucleotide sequence of the CRISPR effector protein, and a guide RNA targeting at least one genome target site (detection target site) or an expression construct comprising a coding nucleotide sequence of the guide RNA targeting at least one genome target site.

In some embodiments, the CRISPR effector protein of the CRISPR detection system of the present invention is a nuclease inactivated CRISPR effector protein. In some embodiments, the CRISPR effector protein of the CRISPR detection system is a CRISPR effector protein with nickase activity. In some embodiments, the CRISPR effector protein of the CRISPR detection system is Cas9 nickase. In some preferred embodiments, the CRISPR effector protein of the CRISPR detection system is a nickase form (nSpCas9) of SpCas9 from *S*. *pyogenes.* For example, the nSpCas9 comprises an amino acid sequence shown in SEQ ID NO: 1. In some embodiments, the CRISPR effector protein of the CRISPR detection system is a nickase form (nSaCas9) of SaCas9 from *S*. *aureus.* For example, the nSaCas9 comprises an amino acid sequence shown in SEQ ID NO: 2.

The incompatibility between the CRISPR detection system and the guide RNA of the base editing system to be detected refers to that the CRISPR detection system cannot use the guide RNA of the base editing system to be detected, and the base editing system to be detected cannot use the guide RNA of the CRISPR detection system. It depends on different CRISPR effector proteins used in the systems.

In some embodiments, the source of the CRISPR effector protein in the CRISPR detection system is different from that of the CRISPR effector protein in the base editor to be detected, so that the guide RNAs of the CRISPR detection system and the base editor to be detected are incompatible.

In some embodiments, the CRISPR effector protein in the CRISPR detection system is derived from SaCas9 of *S*. *aureus,* and the corresponding guide RNA comprises a scaffold sequence shown in SEQ ID NO: 5.

In some embodiments, the CRISPR effector protein in the CRISPR detection system is derived from SpCas9 of *S*. *pyogenes,* and the corresponding guide RNA comprises a scaffold sequence shown in SEQ ID NO: 11.

In some embodiments, the CRISPR effector protein in the base editor the base editor to be detected is derived from SpCas9, for example, is nSpCas9 (SEQ ID NO: 1), and the CRISPR effector protein in the CRISPR detection system is derived from SaCas9, for example, is nSaCas9 (SEQ ID NO: 2).

In some embodiments, the CRISPR effector protein in the CRISPR detection system is derived from SpCas9, for example, is nSpCas9 (SEQ ID NO: 1), and the CRISPR effector protein in the base editor to be detected is derived from SaCas9, for example, is nSaCas9 (SEQ ID NO: 2).

In some embodiments, the CRISPR detection system of the present invention comprises multiple guide RNAs targeting multiple genome detection target sites or an expression construct comprising the coding nucleotide sequences of the multiple guide RNAs. In some embodiments, the base editing system to be detected of the present invention does not comprise a guide RNA or the expression construct thereof, or comprise a guide RNA which targets a site different from the detection target sites of the CRISPR detection system.

In some embodiments, the cell is a eukaryotic cell, such as a mammalian cell or a plant cell. Alternatively, the organism is a eukaryotic organism, such as a mammal or a plant.

In another aspect, the present invention also relates to a kit for use in the method of the present invention. The kit at least comprises the CRISPR detection system of the present invention, and optionally amplification primers of the target sites targeted by the CRISPR detection system.

### Examples

### Example 1. Development of TA-AS system

According to reports, guide RNA frameworks among many CRISPR systems are orthogonal, namely, nuclease in the CRISPR system could only form a protein-RNA complex with the guide RNA of the same system to perform functions. Taking nSpCas9 (Cas9 from *S. pyogenes*, a nickase variant with D10A point mutation) used by a base editing system as an example, the inventor tested whether nSaCas9 (Cas9 from *S*. *aureus,* a nickase variant subjected to D10A point mutation), dSaCas9 (Cas9 from *S*. *aureus,* an inactivated variant with D10A and N580A point mutations) and dLbCpf1 (Cpfl protein of *Lachnospira*, an inactivated variant with D832A point mutation) which were orthogonal to the nSpCas9 could create a single-stranded DNA region for off targets generation by a cytosine base editing system.

### 1.1. Target fragment and vector construction

Table 1 showed target sites of the orthogonal CRISPR systems; PAM sequences were marked in bold; C bases in the target sites were underlined; OsCDC48-SaT1 and OsNRT1.1B-SaT1 target sites were used for testing nSaCas9 and dSaCas9 systems; and OsEPSPS-Cpf1T1 and OsPDS-Cfp1T1 target sites were used for testing the LbCpf1 system.

**Table 1**

| sgRNA | Target sequence | Oligo- F | Oligo- R |
|---|---|---|---|
| *OsCDC48*-SaT1 | CTCGTTCCCATGTCATTGT CATGGGT | GGCGCTCGTTCCCA TGTCATTGTC | AAACGACAATGACAT GGGAACGAG |
| *OsNRT1.1B*-SaT1 | CGATCATCGACAGGTCGG CGGCGGAGT | GGCGCGATCATCG ACAGGTCGGCGG | AAACCCGCCGACCTG TCGATGATCG |
| *OsEPSPS*-Cpf1Tl | TTTATGAAAATATGTATGG AATTCATG | AGATTGAAAATATG TATGGAATTCATG | GGCCCATGAATTCCAT ACATATTTTCA |
| *OsPDS*-Cfp1Tl | TTTGGAGTGAAATCTCTTG TCTTAAGG | AGATGAGTGAAAT CTCTTGTCTTAAGG | GGCCCCTTAAGACAA GAGATTTCACTC |

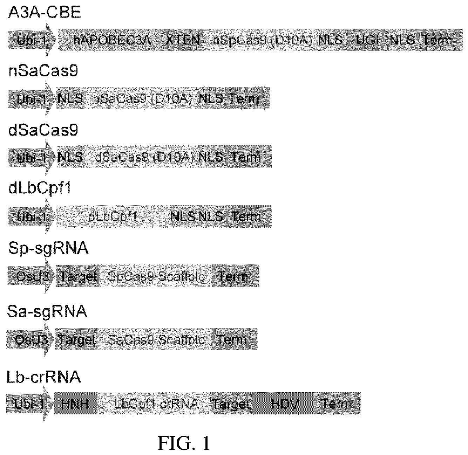

The base editing system to be tested used in this experiment was an A3A-BE3 system, namely the base editor was a fusion protein composed of human APOBEC3A deaminase, nSpCas9 (*S. pyogenes*), UGI (uracil glycosylase inhibitor) and NLS (nuclear localization signal), the expression vector of the fusion protein was pA3A-BE3, and the target vector was pSp-sgRNA. The other three CRISPR systems were pnSaCas9 and pSa-sgRNA, pdSaCas9 and pSa-sgRNA target vectors, pdLbCpf1 and Lb-crRNA, and the vector structures were shown in FIG. 1.

### 1.2. Verification of TA-AS system in rice protoplast transformation

The A3A-BE3 vector was combined with pnSaCas9/pSa-sgRNA-OsCDC48-SaT1, pnSaCas9/pSa-sgRNA-OsNRT1.1B-SaT1, pdSaCas9/pSa-sgRNA-OsCDC48-SaT1, pdSaCas9/pSa-sgRNA-OsNRT1.1B-SaT1, pdLbCpf1/pLb-crRNA-OsEPSPS-Cpf1T1 and pdLbCpf1/pLb-crRNA-OsPDS-Cfp and co-transferred into rice protoplasts.

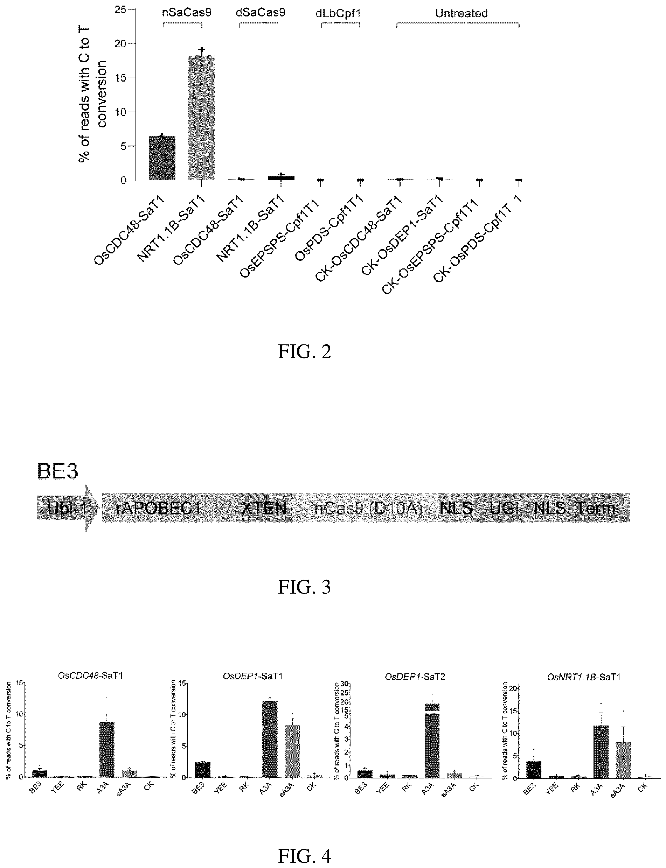

Through amplicon high-throughput sequencing of the target sites, it was found that A3A-BE3 without editing targets had a high-level C-to-T base editing phenomenon on nSaCas9 targeted OsCDC48-SaT1 and OsNRT1.1B-SaT1 target sites, there was no obvious base editing phenomenon detected in other two groups during treatment, and no base editing phenomenon was detected in an untreated group (FIG. 2). It indicated that nSaCas9 could generate a continuous and stable ssDNA region in plants for detecting the random off target effect of the cytosine base editing system in a high-throughput mode.

### Example 2. Detection of off target activity of existing base editing system by amplicon sequencing of target site

The TA-AS system was used for analyzing random off-target effect of reported cytosine base editing systems BE3, YEE-BE3, RK-BE3, A3A-BE3 and eA3A-BE3.

### 2.1. Target fragments and vector construction

The vectors involved in this experiment were the base editing systems based on a BE3 base editor backbone; rAPOBEC1 deaminase in the BE3 vector was replaced with other deaminases to obtain different base editors; the BE3 vector backbone was shown in FIG. 3; RK and YEE represented an R33AK34A variant and a W90YR126ER132E variant of rAPOBEC1 deaminase from a rat; and eA3A represented an N57G variant of human hAPOBEC3A.

The target sites involved in this experiment include target sites in the following Table 2; PAM sequences were marked in bold; C bases in the target sites were underlined; OsAAT1-T1, OsACTG-T1, OsEV-T1 and OsCDC48-T1 were target sites used by the cytosine base editing system; and OsDEP1-SaT1, OsDEP1-SaT2 and OsNRT1.1B-SaT1 were off target detection target sites used by nSaCas9.

**Table 2.**

| sgRNA | Target sequence | Oligo-F | Oligo-R |
|---|---|---|---|
| *OsCDC48*-SaT1 | CTCGTTCCCATGTCATTG TCATGGGT | GGCGCTCGTTCCCATG TCATTGTC | AAACGACAATGACA TGGGAACGAG |
| *OsDEP1-*SaT1 | GGTCACTCAGCCTGCAG | GGCGGGTCACTCAGCC TGCAGTA | AAACTACTGCAGGCT GAGTGACC |
| | TACTGAAT | | |
| *OsDEP1*-SaT2 | GTCGTGCCCTGAATGTT CCTGTGGGT | GGCGGTCGTGCCCTGA ATGTTCCT | AAACAGGAACATTC AGGGCACGAC |
| *OsNRT1.1B*-SaT1 | CGATCATCGACAGGTCG GCGGCGGAGT | GGCGCGATCATCGACA GGTCGGCGG | AAACCCGCCGACCT GTCGATGATCG |

### 2.2. Detection of off target activity of multiple base editing systems by rice protoplast transformation

In this experiment, three vectors, namely different base editing system vectors, a pnSaCas9 vector and a pSa-sgRNA vector, were co-transformed into rice protoplast cells in order to detect the off-target effect of different base editing systems, and the specific efficiency was shown in FIG. 4. The A3A-BE3 system showed the highest random off-target effect, BE3 and eA3A were secondary, and YEE and RK systems hardly have random off-target effect.

### Example 3. Verification of accuracy of TA-AS method by plant individual genome-wide sequencing

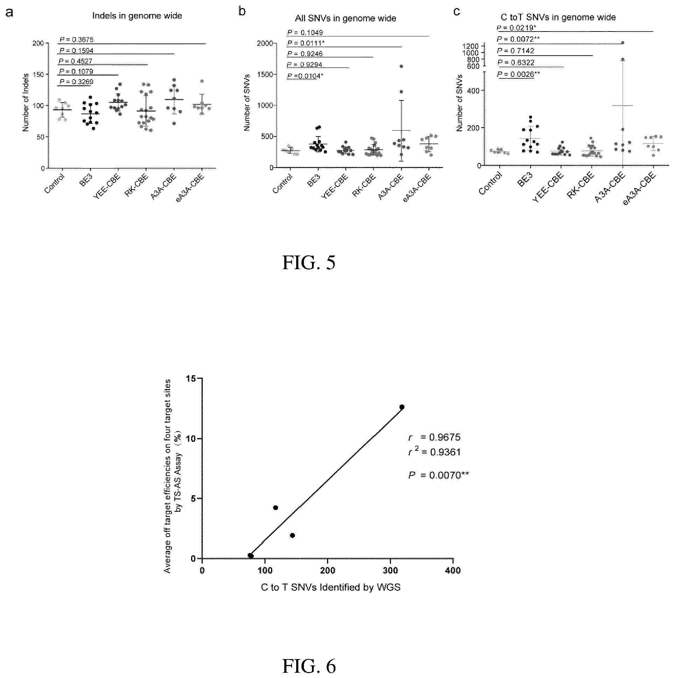

Performing plant genome-wide sequencing to evaluate the genome-wide off-target effect was the most direct and accurate detection method at present. Different base editing system expression vectors were transformed by agrobacterium-mediated transformation to obtain TO-generation regenerated plants of rice over-expressing base editing systems BE3, YEE-BE3, RK-BE3, A3A-BE3 and eA3A-BE3 respectively, and the plants only transformed by agrobacterium were treated as a Control group. The plants were subjected to genome-wide sequencing, and the results showed that there was no significant difference on the number of small fragment insertions and deletions (Indel) in the genome range of the five groups of over-expressing rice (FIG. 5a), but there was significant difference between the BE3 and A3A-BE3 treatment groups and the Control group in the total nucleotide variation number (All SNVs); and compared with the Control group, 102 and 316 additional SNVs were generated respectively (FIG. 5b). For the nucleotide variation number (C to T SNV) of CtoT, the BE3-BE3, A3A-BE3 and eA3A-BE3 had significant difference from the Control group, and 69 and 243 additional C to T SNVs were generated respectively (FIG. 5C). On the contrary, there was no obvious off target phenomenon detected from YEE-BE3 and RK-BE3 (FIG. 5). In addition, it was found from the correlation analysis of the average value of the C to T SNVs of the five base editing system treatment groups in this experiment and the off-target effect detected by the TA-AS system in FIG. 4 that the TA-AS method had significant correlation with the genome-wide sequencing result (FIG. 6). For the five base editing systems, the TA-AS method and the genome-wide sequencing method have the same experimental results, which indicated that the method had high sensitivity and accuracy, and could be used for simply detecting the random off-target effect of the base editing system in a high-throughput mode.

### Sequence Listing

SEQ ID NO: 1 nSpCas9 amino acid sequence 
DKKYSIGLAIGTNSVGWAVITDEYKVPSKKFKVLGNTDRHSIKKNLIGALLFDSGETAEATRLK
RTARRRYTRRKNRICYLQEIFSNEMAKVDDSFFHRLEESFLVEEDKKHERHPIFGNIVDEVAYHE
KYPTIYHLRKKLVDSTDKADLRLIYLALAHMIKFRGHFLIEGDLNPDNSDVDKLFIQLVQTYNQ
LFEENPINASGVDAKAILSARLSKSRRLENLIAQLPGEKKNGLFGNLIALSLGLTPNFKSNFDLA
EDAKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKMDGTE
ELLVKLNREDLLRKQRTFDNGSIPHQIHLGELHAILRRQEDFYPFLKDNREKIEKILTFRIPYYVG
PLARGNSRFAWMTRKSEETITPWNFEEVVDKGASAQSFIERMTNFDKNLPNEKVLPKHSLLYE
YFTVYNELTKVKYVTEGMRKPAFLSGEQKKAIVDLLFKTNRKVTVKQLKEDYFKKIECFDSVE
ISGVEDRFNASLGTYHDLLKIIKDKDFLDNEENEDILEDIVLTLTLFEDREMIEERLKTYAHLFDD
KVMKQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMARENQTTQ
KGQKNSRERMKRIEEGIKELGSQILKEHPVENTQLQNEKLYLYYLQNGRDMYVDQELDINRLS
DYDVDHIVPQSFLKDDSIDNKVLTRSDKNRGKSDNVPSEEVVKKMKNYWRQLLNAKLITQRK
FDNLTKAERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVYDVRKMI
AKSEQEIGKATAKYFFYSNIMNFFKTEITLANGEIRKRPLIETNGETGEIVWDKGRDFATVRKVL
SMPQVNIVKKTEVQTGGFSKESILPKRNSDKLIARKKDWDPKKYGGFDSPTVAYSVLVVAKVE
KGKSKKLKSVKELLGITIMERSSFEKNPIDFLEAKGYKEVKKDLIIKLPKYSLFELENGRKRML
ASAGELQKGNELALPSKYVNFLYLASHYEKLKGSPEDNEQKQLFVEQHKHYLDEIIEQISEFSK
RVILADANLDKVLSAYNKHRDKPIREQAENIIHLFTLTNLGAPAAFKYFDTTIDRKRYTSTKEVL
DATLIHQSITGLYETRIDLSQLGGDKRPAATKKAGQAKKKK
SEQ ID NO: 2 nSaCas9 amino acid sequence 
KRNYILGLAIGITSVGYGIIDYETRDVIDAGVRLFKEANVENNEGRRSKRGARRLKRRRRHRIQ
RVKKLLFDYNLLTDHSELSGINPYEARVKGLSQKLSEEEFSAALLHLAKRRGVHNVNEVEEDT
GNELSTKEQISRNSKALEEKYVAELQLERLKKDGEVRGSINRFKTSDYVKEAKQLLKVQKAYH
QLDQSFIDTYIDLLETRRTYYEGPGEGSPFGWKDIKEWYEMLMGHCTYFPEELRSVKYAYNAD
LYNALNDLNNLVITRDENEKLEYYEKFQIIENVFKQKKKPTLKQIAKEILVNEEDIKGYRVTSTG
KPEFTNLKVYHDIKDITARKEIIENAELLDQIAKILTIYQSSEDIQEELTNLNSELTQEEIEQISNLK
GYTGTHNLSLKAINLILDELWHTNDNQIAIFNRLKLVPKKVDLSQQKEIPTTLVDDFILSPVVKR
 
SFIQSIKVINAIIKKYGLPNDIIIELAREKNSKDAQKMINEMQKRNRQTNERIEEIIRTTGKENAK
YLIEKIKLHDMQEGKCLYSLEAIPLEDLLNNPFNYEVDHIIPRSVSFDNSFNNKVLVKQEENSKK
GNRTPFQYLSSSDSKISYETFKKHILNLAKGKGRISKTKKEYLLEERDINRFSVQKDFINRNLVD
TRYATRGLMNLLRSYFRVNNLDVKVKSINGGFTSFLRRKWKFKKERNKGYKHHAEDALIIAN
ADFIFKEWKKLDKAKKVMENQMFEEKQAESMPEIETEQEYKEIFITPHQIKHIKDFKDYKYSHR
VDKKPNRELINDTLYSTRKDDKGNTLIVNNLNGLYDKDNDKLKKLINKSPEKLLMYHHDPQT
YQKLKLIMEQYGDEKNPLYKYYEETGNYLTKYSKKDNGPVIKKIKYYGNKLNAHLDITDDYP
NSRNKVVKLSLKPYRFDVYLDNGVYKFVTVKNLDVIKKENYYEVNSKCYEEAKKLKKISNQ
AEFIASFYNNDLIKINGELYRVIGVNNDLLNRIEVNMIDITYREYLENMNDKRPPRIIKTIASKTQ
SIKKYSTDILGNLYEVKSKKHPQIIKKG
SEQ ID NO: 3 dSaCas9 amino acid sequence 
KRNYILGLAIGITSVGYGIIDYETRDVIDAGVRLFKEANVENNEGRRSKRGARRLKRRRRHRIQ
RVKKLLFDYNLLTDHSELSGINPYEARVKGLSQKLSEEEFSAALLHLAKRRGVHNVNEVEEDT
GNELSTKEQISRNSKALEEKYVAELQLERLKKDGEVRGSINRFKTSDYVKEAKQLLKVQKAYH
QLDQSFIDTYIDLLETRRTYYEGPGEGSPFGWKDIKEWYEMLMGHCTYFPEELRSVKYAYNAD
LYNALNDLNNLVITRDENEKLEYYEKFQIIENVFKQKKKPTLKQIAKEILVNEEDIKGYRVTSTG
KPEFTNLKVYHDIKDITARKEIIENAELLDQIAKILTIYQSSEDIQEELTNLNSELTQEEIEQISNLK
GYTGTHNLSLKAINLILDELWHTNDNQIAIFNRLKLVPKKVDLSQQKEIPTTLVDDFILSPVVKR
SFIQSIKVINAIIKKYGLPNDIIIELAREKNSKDAQKMINEMQKRNRQTNERIEEIIRTTGKENAK
YLIEKIKLHDMQEGKCLYSLEAIPLEDLLNNPFNYEVDHIIPRSVSFDNSFNNKVLVKQEEASKK
GNRTPFQYLSSSDSKISYETFKKHILNLAKGKGRISKTKKEYLLEERDINRFSVQKDFINRNLVD
TRYATRGLMNLLRSYFRVNNLDVKVKSINGGFTSFLRRKWKFKKERNKGYKHHAEDALIIAN
ADFIFKEWKKLDKAKKVMENQMFEEKQAESMPEIETEQEYKEIFITPHQIKHIKDFKDYKYSHR
VDKKPNRELINDTLYSTRKDDKGNTLIVNNLNGLYDKDNDKLKKLINKSPEKLLMYHHDPQT
YQKLKLIMEQYGDEKNPLYKYYEETGNYLTKYSKKDNGPVIKKIKYYGNKLNAHLDITDDYP
NSRNKVVKLSLKPYRFDVYLDNGVYKFVTVKNLDVIKKENYYEVNSKCYEEAKKLKKISNQ
AEFIASFYNNDLIKINGELYRVIGVNNDLLNRIEVNMIDITYREYLENMNDKRPPRIIKTIASKTQ
SIKKYSTDILGNLYEVKSKKHPQIIKKG
SEQ ID NO: 4 dLbCpf1 amino acid sequence 
MSKLEKFTNCYSLSKTLRFKAIPVGKTQENIDNKRLLVEDEKRAEDYKGVKKLLDRYYLSFIN
DVLHSIKLKNLNNYISLFRKKTRTEKENKELENLEINLRKEIAKAFKGNEGYKSLFKKDIIETILP
EFLDDKDEIALVNSFNGFTTAFTGFFDNRENMFSEEAKSTSIAFRCINENLTRYISNMDIFEKVDA
IFDKHEVQEIKEKILNSDYDVEDFFEGEFFNFVLTQEGIDVYNAIIGGFVTESGEKIKGLNEYINL
YNQKTKQKLPKFKPLYKQVLSDRESLSFYGEGYTSDEEVLEVFRNTLNKNSEIFSSIKKLEKLF
 
KNFDEYSSAGIFVKNGPAISTISKDIFGEWNVIRDKWNAEYDDIHLKKKAVVTEKYEDDRRKSF
KKIGSFSLEQLQEYADADLSVVEKLKEIIIQKVDEIYKVYGSSEKLFDADFVLEKSLKKNDAVV
AIMKDLLDSVKSFENYIKAFFGEGKETNRDESFYGDFVLAYDILLKVDHIYDAIRNYVTQKPYS
KDKFKLYFQNPQFMGGWDKDKETDYRATILRYGSKYYLAIMDKKYAKCLQKIDKDDVNGNY
EKINYKLLPGPNKMLPKVFFSKKWMAYYNPSEDIQKIYKNGTFKKGDMFNLNDCHKLIDFFK
DSISRYPKWSNAYDFNFSETEKYKDIAGFYREVEEQGYKVSFESASKKEVDKLVEEGKLYMFQI
YNKDFSDKSHGTPNLHTMYFKLLFDENNHGQIRLSGGAELFMRRASLKKEELVVHPANSPIAN
KNPDNPKKTTTLSYDVYKDKRFSEDQYELHIPIAINKCPKNIFKINTEVRVLLKHDDNPYVIGIA
RGERNLLYIVVVDGKGNIVEQYSLNEIINNFNGIRIKTDYHSLLDKKEKERFEARQNWTSIENIK
ELKAGYISQVVHKICELVEKYDAVIALEDLNSGFKNSRVKVEKQVYQKFEKMLIDKLNYMVD
KKSNPCATGGALKGYQITNKFESFKSMSTQNGFIFYIPAWLTSKIDPSTGFVNLLKTKYTSIADS
KKFISSFDRIMYVPEEDLFEFALDYKNFSRTDADYIKKWKLYSYGNRIRIFRNPKKNNVFDWEE
VCLTSAYKELFNKYGINYQQGDIRALLCEQSDKAFYSSFMALMSLMLQMRNSITGRTDVDFLI
SPVKNSDGIFYDSRNYEAQENAILPKNADANGAYNIARKVLWAIGQFKKAEDEKLDKVKIAIS
NKEWLEYAQTSVKH
SEQ ID NO: 5 Sa-sgRNA scaffold sequence 
GUUUUAGUACUCUGGAAACAGAAUCUACUAAAACAAGGCAAAAUGCCGUGUUUAUCUC
GUCAACUUGUUGGCGAGA
SEQ ID NO: 6 Lb-crRNA scaffold sequence
   UAAUUUCUACUaagUGUAGAU
SEQ ID NO: 7 hA3A deaminase 
EASPASGPRHLMDPHIFTSNFNNGIGRHKTYLCYEVERLDNGTSVKMDQHRGFLHNQAKNLL
CGFYGRHAELRFLDLVPSLQLDPAQIYRVTWFISWSPCFSWGCAGEVRAFLQENTHVRLRIFAA
RIYDYDPLYKEALQMLRDAGAQVSIMTYDEFKHCWDTFVDHQGCPFQPWDGLDEHSQALSG
RLRAILQNQGNSGSETPGTSESATPES
SEQ ID NO: 8 rAPOBEC1-RK deaminase 
SSETGPVAVDPTLRRRIEPHEFEVFFDPRELAAETCLLYEINWGGRHSIWRHTSQNTNKHVEVN
FIEKFTTERYFCPNTRCSITWFLSWSPCGECSRAITEFLSRYPHVTLFIYIARLYHHADPRNRQGL
RDLISSGVTIQIMTEQESGYCWRNFVNYSPSNEAHWPRYPHLWVRLYVLELYCIILGLPPCLNIL
RRKQPQLTFFTIALQSCHYQRLPPHILWATGLK
SEQ ID NO: 9 rAPOBEC1-YEE deaminase 
SSETGPVAVDPTLRRRIEPHEFEVFFDPRELRKETCLLYEINWGGRHSIWRHTSQNTNKHVEVN
FIEKFTTERYFCPNTRCSITWFLSYSPCGECSRAITEFLSRYPHVTLFIYIARLYHHADPENRQGLE
DLISSGVTIQIMTEQESGYCWRNFVNYSPSNEAHWPRYPHLWVRLYVLELYCIILGLPPCLNILR
 RKQPQLTFFTIALQSCHYQRLPPHILWATGLK
SEQ ID NO: 10 rAPOBEC1-eA3A deaminase 
EASPASGPRHLMDPHIFTSNFNNGIGRHKTYLCYEVERLDNGTSVKMDQHRGFLHGQAKNLL
CGFYGRHAELRFLDLVPSLQLDPAQIYRVTWFISWSPCFSWGCAGEVRAFLQENTHVRLRIFAA
RIYDYDPLYKEALQMLRDAGAQVSIMTYDEFKHCWDTFVDHQGCPFQPWDGLDEHSQALSG
RLRAILQNQGNSGSETPGTSESATPES
SEQ ID NO: 11 SpsgRNA scaffold sequence

GUUUUAGAGCUAGAAAUAGCAAGUUAAAAUAAGGCUAGUCCGUUAUCAACUUGAAAAA
GUGGCACCGAGUCGGUGC

## Claims

1. A method for detecting the random off-target effect of a base editing system, comprising:
a) introducing a base editing system to be detected into a cell or an organism;
b) introducing a CRISPR detection system which targets at least one detection target site in the genome into the cell or the organism, wherein the CRISPR detection system being capable of forming a single-stranded DNA region in the at least one detection target site, and the guide RNA of the CRISPR detection system being incompatible with the guide RNA of the base editing system to be detected;
c) extracting nucleic acid from the cell or the organism, amplifying the sequence of the at least one detection target site, and sequencing the amplicons; and
d) determining nucleotide mutation in the at least one detection target site.

2. The method according to claim 1, wherein the base editing system to be detected comprises a base editor to be detected or an expression construct comprising a coding sequence thereof, and/or a corresponding guide RNA (gRNA) or an expression construct comprising a coding sequence thereof.

3. The method according to claim 1, wherein the base editing system to be detected comprises a cytosine base editor.

4. The method according to claim 3, wherein the cytosine base editor is a fusion protein containing a CRISPR effector protein and a cytosine deaminase.

5. The method according to claim 4, wherein the cytosine deaminase is selected from the group consisting of APOBEC1 deaminase, activation-induced cytidine deaminase (AID), APOBEC3G, CDA1, human APOBEC3A deaminase, or functional variants thereof, for example, the cytosine deaminase comprises an amino acid sequence of one of SEQ ID NOs: 7-10.

6. The method according to claim 4, wherein the CRISPR effector protein of the base editor is a nuclease inactivated CRISPR effector protein, such as a CRISPR effector protein with nickase activity.

7. The method according to claim 4, wherein the CRISPR effector protein of the base editor is Cas9 nickase.

8. The method according to claim 4, wherein the CRISPR effector protein of the base editor is a nickase form (nSpCas9) of SpCas9 from *S*. *pyogenes,* for example, the nSpCas9 comprises an amino acid sequence shown in SEQ ID NO: 1.

9. The method according to claim 4, wherein the CRISPR effector protein of the base editor is a nickase form (nSaCas9) of SaCas9 from *S*. *aureus,* for example, the nSaCas9 comprises an amino acid sequence shown in SEQ ID NO: 2.

10. The method according to any one of claims 1 to 9, wherein the CRISPR detection system comprises a CRISPR effector protein or an expression construct comprising a coding nucleotide sequence thereof, and a corresponding guide RNA targeting at least one genome detection target site or an expression construct comprising a coding nucleotide sequence thereof.

11. The method according to claim 10, wherein the CRISPR effector protein of the CRISPR detection system is a nuclease inactivated CRISPR effector protein, such as a CRISPR effector protein with nickase activity.

12. The method according to claim 10, wherein the CRISPR effector protein of the CRISPR detection system is Cas9 nickase.

13. The method according to claim 10, wherein the CRISPR effector protein of the CRISPR detection system is a nickase form (nSpCas9) of SpCas9 from *S. pyogenes*, for example, the nSpCas9 comprises an amino acid sequence shown in SEQ ID NO: 1.

14. The method according to claim 10, wherein the CRISPR effector protein of the CRISPR detection system is a nickase form (nSaCas9) of SaCas9 from *S*. *aureus,* for example, the nSaCas9 comprises an amino acid sequence shown in SEQ ID NO: 2.

15. The method according to any one of claims 1 to 14, wherein the CRISPR effector protein in the CRISPR detection system is derived from a source different from that of the CRISPR effector protein in the base editor to be detected, so that the guide RNAs of the CRISPR detection system and the base editor to be detected are incompatible.

16. The method according to any one of claims 1 to 15, wherein the CRISPR effector protein in the base editor is derived from SpCas9, such as nSpCas9 (SEQ ID NO: 1), and the CRISPR effector protein in the CRISPR detection system is derived from SaCas9, such as nSaCas9 (SEQ ID NO: 2).

17. The method according to any one of claims 1 to 15, wherein the CRISPR effector protein in the CRISPR detection system is derived from SpCas9, such as nSpCas9 (SEQ ID NO: 1), and the CRISPR effector protein in the base editor is derived from SaCas9, such as nSaCas9 (SEQ ID NO: 2).

18. The method according to any one of claims 1 to 17, wherein the CRISPR detection system comprises a plurality of guide RNAs targeting a plurality of genome detection target sites or an expression construct comprising coding nucleotide sequences thereof.

19. The method according to any one of claims 1 to 18, wherein the base editing system to be detected does not comprises a guide RNA or an expression construct thereof, or comprises a guide RNA which targets a site different from the detection target sites of the CRISPR detection system.

20. The method according to any one of claims 1 to 19, wherein the cell is a eukaryotic cell, such as a mammalian cell or a plant cell; or, the organism is a eukaryotic organism, such as a mammal or a plant.
